# EUROPÄISCHE PATENTSCHRIFT

(11) **EP 2 799 039 B1**
(45) Veröffentlichungstag und Bekanntmachung des Hinweises auf die Patenterteilung: **17.10.2018**
(21) Anmeldenummer: 14164825.3
(22) Anmeldetag: 16.04.2014
(51) Int. Cl.: A61F 5/01, A61F 5/04, A61F 5/042

(54) **Orthese**
Orthotic
Orthèse

(30) Priorität: 03.05.2013 DE 102013208165
(43) Veröffentlichungstag der Anmeldung: 05.11.2014
(73) Patentinhaber: Pohlig GmbH, 83278 Traunstein (DE)
(72) Erfinder: Pohlig, Kurt, 83278 Traunstein (DE); Pohlig-Wetzelsperger, Claudia, 83278 Traunstein (DE)
(74) Vertreter: Pfenning, Meinig & Partner mbB

(56) Entgegenhaltungen:
- EP-A1- 2 179 712
- EP-A1- 2 540 259
- WO-A1-00/18337
- US-B1- 8 343 083

## Beschreibung

Die Erfindung betrifft eine Orthese zur Entlastung von Körpergelenken sowie zur Behandlung von Gelenkfehlstellungen und/oder Gelenkerkrankungen.

Die EP 2 179 712 A1 beschreibt eine Orthese zum gestreckten Halten, insbesondere Auseinanderziehen, eines Sprunggelenks. Das auseinandergezogene Halten eines Gelenks und die damit erreichte Entlastung des Gelenks sind vorteilhaft bei vielen Behandlungsverfahren und Operationstechniken bei Gelenkerkrankungen und Gelenkfehlstellungen. Die beschriebene Orthese weist eine Unterschenkelschale zum Fixieren der Orthese an einem Unterschenkel und ein Fußteil zum Fixieren der Orthese an einem Fuß auf. Unterschenkelschale und Fußteil sind dabei über Schienen miteinander verbunden, die in einem Distraktionsschlittensystem geführt werden. Bei angelegter Orthese können mittels des Schlittensystems die Unterschenkelschale und das Fußteil auseinandergezogen werden. Hierbei stützt sich die Unterschenkelschale an einer knöchernen Struktur des Schienbeins ab, wodurch sich eine Distraktion des Sprunggelenks ergibt.

Eine Abstützung der Unterschenkelschale bzw. des Fußteils am Knochen kann in vielen Fällen und besonders über einen längeren Zeitraum sehr schmerzhaft sein. Ferner ist eine Abstützung an den Knochen zur Distraktion nicht für alle Gelenkarten geeignet, wenn die am Gelenk beteiligten Knochen keine ausreichende Abstützfläche bieten. Sind keine ausreichenden Abstützflächen vorhanden, kann die Orthese verrutschen und so keine Distraktionskraft entfalten. Beispielsweise nicht bekannt, wie eine Abstützung oberhalb eines Kniegelenks für eine effektive Distraktion des Kniegelenks realisierbar ist.

Ferner ist aus dem Stand der Technik ein äußerer Fixierungsrahmen zur Behandlung von Gelenkfehlstellungen, beispielsweise am Knie, bekannt. Hier werden Pins in die am Gelenk beteiligten Knochen beidseits des Gelenks getrieben und dort verankert. Diese Pins werden von außen üblicherweise durch längenverstellbare Stangen miteinander verbunden, die eine Variation des Abstands der Pins beidseits des Gelenkspalts ermöglichen. Durch eine schrittweise Verlängerung dieser Verbindungsstangen ergibt sich eine Vergrößerung des Gelenkspalts. In dieser Haltung kann das Gelenk dann auf schonende Art und Weise in eine Sollstellung gebracht werden.

Eine solche invasive Gelenkdistraktion ist auch aus "Tissue structure modification in knee osteoarthritis by use of joint distraction: an open 1-year pilot study", Femke Intema et al., Ann Rheum Dis 2011; 70, 1441-1446. doi:10.1136/ard.2010142364 zur Behandlung von Arthrose bekannt.

Nachteilig an der Behandlung mittels eines äußeren Fixierungsrahmens ist jedoch, dass hierzu stets ein operativer Eingriff mit den damit einhergehenden nicht unerheblichen Operationsrisiken notwendig ist.

Ausgehend vom Stand der Technik ist es daher die Aufgabe der Erfindung, eine Orthese und ein Verfahren zum Herbeiführen und Halten einer auseinandergezogenen Stellung eines Körpergelenks, im Folgenden auch "Gelenk" genannt, bereitzustellen, wobei eine effektive Distraktion des Gelenks ohne einen operativen Eingriff in das Körpergewebe möglich ist.

Die Aufgabe wird durch eine Orthese nach Anspruch 1 gelöst. Weitere vorteilhafte Ausgestaltungen der erfindungsgemäßen Orthese sind in den abhängigen Ansprüchen 2 bis 13 beschrieben.

Die nachfolgend beschriebene Erfindung ermöglicht eine Fixierung eines Gelenks in einer vorgegebenen Stellung ohne die Vornahme von operativen Eingriffen in das Körpergewebe. Insbesondere ist durch die Erfindung ein Herbeiführen einer zumindest zeitweise entlasteten Stellung und/oder einer auseinandergezogenen Stellung (Distraktion) eines Gelenks sowie das Halten des Gelenks in einer derartigen Stellung möglich.

Erfindungsgemäß weist die Orthese zum Herbeiführen einer zumindest zeitweise entlasteten und/oder auseinandergezogenen Stellung eines Körpergelenks mindestens ein erstes Halteteil zur Aufnahme eines ersten Körperteils und ein zweites Halteteil zur Aufnahme eines mit dem ersten Körperteil über das Körpergelenk verbundenen zweiten Körperteils auf. Das erste Halteteil weist dabei mindestens einen ersten Abstützbereich auf, in dem das erste Halteteil geeignet ist, sich in einer Längsrichtung des ersten Halteteils und vom zweiten Halteteil weg gerichtet am ersten Körperteil abzustützen. Des Weiteren weist das zweite Halteteil mindestens einen zweiten Abstützbereich auf, in dem das zweite Halteteil geeignet ist, sich in einer Längsrichtung des zweiten Halteteils und vom ersten Halteteil weg gerichtet am zweiten Körperteil abzustützen.

Ferner umfasst die erfindungsgemäße Orthese eine Stellvorrichtung, über die der erste Abstützbereich mit dem zweiten Abstützbereich kraftschlüssig verbunden ist und die geeignet ist, auf den ersten und den zweiten Abstützbereich jeweils entgegengesetzte Schubkräfte auszuüben. Mit anderen Worten ist die Stellvorrichtung dazu eingerichtet, jeweils auf den ersten Abstützbereich eine vom zweiten Abstützbereich weg gerichtete Kraft und auf den zweiten Abstützbereich eine vom ersten Abstützbereich weg gerichtete Kraft auszuüben, mit dem Ziel den Abstand zwischen dem ersten und dem zweiten Halteteil zu vergrößern. Die Abstützbereiche übertragen dabei die auf sie durch die Stellvorrichtung ausgeübte Kraft auf die durch das Gelenk miteinander verbundenen Knochen. Ist die Kraft der Stellvorrichtung gleich der oder größer als die das Gelenk zusammenhaltende Kraft (Kraft des Körpergewebes (Muskeln, Bänder, usw.), Gewichtskraft des oberen Körperteils), so stabilisiert oder vergrößert sich der Abstand zwischen den durch das Gelenk miteinander verbundenen Knochen, wodurch das Gelenk in einer vorgegebenen Weise entlastet und/oder auseinandergezogen (Distraktion) wird.

Die erfindungsgemäße Orthese zeichnet sich dadurch aus, dass der erste und/oder der zweite Abstützbereich auf einer Körperkontaktseite des ersten bzw. zweiten Halteteils, also auf der dem von dem Halteteil aufgenommenen Körperteil zugewandten Innenseite des ersten bzw. zweiten Halteteils, eine oder mehrere Ausnehmungen aufweisen, so dass das erste und/oder das zweite Halteteil geeignet sind, sich in Längsrichtung an einem Gewebe des ersten und/oder des zweiten Körperteils abzustützen. Während des Tragens der Orthese schiebt sich das von einer Ausnehmung überdeckte Gewebe durch die Ausnehmung nach außen. Das verformte Gewebe bildet so eine Abstützfläche für das Halteteil, an der sich eine dem jeweils anderen Halteteil zugewandte oder dem Körpergelenk zugewandte Kante der Ausnehmung abstützen kann, wenn auf den die Ausnehmung enthaltenen Abstützbereich eine Schubkraft aus Richtung des jeweils anderen Halteteils oder aus Richtung des Körpergelenks ausgeübt wird.

In einer bevorzugten Ausführungsform der Erfindung können die Ausnehmungen als Löcher oder Durchgangslöcher mit vorzugsweise konvex geformten Innenkanten oder Ausbuchtungen ausgebildet sein.

Weisen das erste und/oder das zweite Halteteil mehrere Ausnehmungen auf, so sind die Ausnehmungen vorzugsweise entlang eines Umfangs der Halteteile angeordnet. Insbesondere ist es vorteilhaft, wenn die Ausnehmungen entlang von Muskeln oder Bereichen mit stützendem Gewebe des ersten und/oder zweiten Körperteils am ersten bzw. zweiten Halteteil angeordnet sind.

Besonders bevorzugt sind die Ausnehmungen in einem Schnitt parallel zu einer Oberfläche der Halteteile länglich, oval und/oder konisch geformt ausgebildet, wobei die Längsachsen der Ausnehmungen parallel zur Längsrichtung der Halteteile ausgerichtet sind.

Die erfindungsgemäße Orthese eignet sich vorzugsweise zur Distraktion eines Kniegelenks. Dazu sind das erste Halteteil zur Aufnahme eines Oberschenkels und/oder eines Rumpfes/Beckens und das zweite Halteteil zur Aufnahme eines Unterschenkels und/oder Fußes geeignet. Mittels der Orthese kann dann eine zumindest zeitweise entlastete und/oder auseinandergezogene Stellung eines Kniegelenks herbeigeführt werden.

In einer weiteren bevorzugten Ausgestaltung der Erfindung ist der zweite Abstützbereich derart ausgebildet, dass das zweite Halteteil geeignet ist, sich an einer prominenten Stelle eines Unterschenkelknochens im Bereich eines Sprunggelenks und/oder an einem Fußrücken abzustützen. Unter einer prominenten Stelle eines Unterschenkelknochens wird ein im Bereich des Sprunggelenks senkrecht zur Längsrichtung des Unterschenkels hervorstehender Teil eines am Sprunggelenk beteiligten Knochens bzw. eine Verbreiterung an einem Ende eines Unterschenkelknochens im Bereich des Sprunggelenks verstanden.

Stützt sich das zweite Halteteil mit dem zweiten Abstützbereich am Fußrücken ab, kann mit der Entlastung bzw. Distraktion des Kniegelenks auch eine zumindest teilweise Entlastung bzw. Distraktion des Sprunggelenks eintreten. Eine derartig ausgebildete Orthese ermöglicht also eine gleichzeitige Behandlung des Knie- und Sprunggelenks. Erfolgt lediglich eine Abstützung an einer prominenten Stelle des Unterschenkelknochens, kommt es zu keiner Entlastung bzw. Distraktion im Sprunggelenk. Eine Abstützung am Fußrücken ist besonders bei der Therapie einer Pseudoarthrose oder einem Pseudoarthrose-ähnlichen Zustand günstig. Ein weiterer Vorteil einer Abstützung sowohl am Unterschenkelknochen als auch am Fußrücken liegt darin, dass sich der durch die Abstützung ausgeübte Druck auf eine größere Körperfläche verteilt, wodurch sich der Tragekomfort für den Patienten erhöht. Ferner kann dadurch die von der Stellvorrichtung ausgeübte Kraft effektiver auf das Körperskelett übertragen werden.

Alternativ kann der zweite Abstützbereich des zweiten Halteteils auch derart ausgebildet sein, dass das zweite Halteteil geeignet ist, sich an einer Wade abzustützen. In diesem Fall befindet sich der zweite Abstützbereich in einem dem ersten Halteteil zugewandten Bereich des zweiten Halteteils.

Ferner ist es auch denkbar, dass der zweite Abstützbereich zweiteilig ausgebildet ist, das heißt, einen unteren zweiten und einen oberen zweiten Teilbereich zur Abstützung in zwei verschiedenen Bereichen längs des Unterschenkels und/oder am Fuß aufweist. Dies ermöglicht beispielsweise eine Abstützung des zweiten Halteteils sowohl an der Wade als auch an einer prominenten Stelle des Unterschenkelknochens im Bereich des Sprunggelenks oder am Fußrücken. Auch bei einem zweiteiligen zweiten Abstützbereich ist die Stellvorrichtung mit dem gesamten zweiten Abstützbereich, das heißt sowohl mit dem unteren zweiten Teilbereich als auch mit dem oberen zweiten Teilbereich, kraftschlüssig verbunden. Die Nutzung mehrerer Abstützbereiche am zweiten Halteteil verbessert die Kraftübertragung der Stellvorrichtung über den zweiten Abstützbereich des zweiten Halteteils auf den Unterschenkel und den Fuß und ermöglicht so eine stärkere und präziser einzustellende Entlastung bzw. Distraktion des Kniegelenks und/oder Sprunggelenks. Ferner wird allgemein bei einer größeren Anzahl an Abstützbereichen der Druck auf die entsprechenden Körperstellen vermindert, was wiederum den Komfort für den Patienten erhöht.

Des Weiteren kann das zweite Halteteil eine Unterschenkelhülse und eine Fußfassung aufweisen, welche zueinander beweglich oder starr miteinander verbunden sind. In diesem Fall ist es denkbar, dass sich ein oberer zweiter Abstützbereich an der Unterschenkelhülse zur Abstützung an der Wade oder zur Abstützung an einer prominenten Stelle des Unterschenkelknochens befindet. Ein unterer zweiter Abstützbereich kann dann an der Fußfassung zur Abstützung am Fußrücken vorgesehen sein. Ferner erhöht sich durch die Verwendung einer Unterschenkelhülse in Kombination mit einer Fußfassung die Rotationsstabilität der Orthese.

Die Fußfassung und die Unterschenkelhülse können außerdem vorzugsweise über ein Orthesengelenk um eine Achse des Orthesengelenks zueinander drehbar verbunden sein. Dies erhöht die Beweglichkeit der Orthese und damit die Bewegungsfreiheit des Patienten bei gleichzeitig effektiver Kraftübertragung durch zwei Abstützbereiche am zweiten Halteteil.

Besonders bevorzugt ist die Fußfassung als Ringfassung ausgebildet. Durch eine Ringfassung erhöht sich die Rotationsstabilität der Orthese. Ferner werden Unterschenkel und Fuß in einer Unterschenkelhülse mit zusätzlicher Ringfassung dreidimensional derart fixiert, dass eine definierte Entlastungs- und/oder Distraktionswirkung auf das Knie- und/oder Sprunggelenk erreicht wird.

Anstelle einer Fußfassung kann das zweite Halteteil auch eine Unterschenkelhülse aufweisen, die in ihrem dem ersten Halteteil abgewandten Bereich eine Abstützplatte zur Abstützung am Fußrücken aufweist. Mit einer derartigen Unterschenkelhülse mit Abstützplatte in Verbindung mit einem Schuh kann eine ähnliche Wirkung wie bei der Ringfassung erreicht werden. Der Vorteil einer Abstützplatte gegenüber der Ringfassung ist ein geringeres Gewicht der Orthese sowie ein geringerer Materialaufwand. Ferner ist es möglich die Orthese mit handelsüblichen Schuhen zu Tragen, was wiederum den Tragekomfort für den Patienten erhöht.

In einer weiteren vorteilhaften Ausgestaltung der Erfindung ist der erste Abstützbereich des ersten Halteteils derart ausgebildet, dass das erste Halteteil geeignet ist, den Oberschenkel in der Nähe des Kniegelenks weitgehend zu umgreifen und sich dadurch an mindestens einer prominenten Stelle eines Oberschenkelknochens abzustützen. Ferner ist es auch denkbar, dass sich der erste Abstützbereich zur Abstützung des ersten Halteteils an einem Sitzbein eignet. Unter einer prominenten Stelle eines Oberschenkelknochens wird ein radial aus einem in etwa zylinderförmigen Oberschenkelknochen hervorstehender Knochenteil im Bereich des Kniegelenks oder eine Verbreiterung eines Oberschenkelknoches im Bereich des Kniegelenks verstanden.

Besonders vorteilhaft ist es, wenn der erste Abstützbereich zweiteilig gestaltet ist. In diesem Fall weist der erste Abstützbereich einen oberen ersten Teilbereich und einen unteren ersten Teilbereich zur Abstützung in zwei verschiedenen Bereichen längs des Oberschenkels und/oder am Sitzbein auf. Beispielsweise ist der untere erste Teilbereich derart am ersten Halteteil ausgebildet, dass das erste Halteteil in dem unteren ersten Teilbereich geeignet ist, den Oberschenkel in der Nähe des Kniegelenks weitgehend zu umgreifen und sich dadurch an mindestens einer prominenten Stelle eines Oberschenkelknochens, also an einem senkrecht zur Längsrichtung eines Oberschenkels hervorstehenden Knochenteils des Oberschenkelknochens, im Bereich des Kniegelenks abzustützen. Auf seiner dem unteren ersten Teilbereich abgewandten Seite kann das erste Halteteil dann zusätzlich einen oberen ersten Teilbereich aufweisen, in dem das Halteteil geeignet ist, sich am Sitzbein abzustützen. Alternativ kann der obere erste Teilbereich auch derart ausgebildet sein, dass sich das Halteteil im oberen ersten Teilbereich lediglich am Gewebe des Oberschenkels, wie z.B. an den Muskeln, abstützen kann. In diesem Fall weist der obere erste Teilbereich Ausnehmungen zur Abstützung am Gewebe auf. Auch bei einem zweiteiligen ersten Abstützbereich ist die Stellvorrichtung mit dem gesamten ersten Abstützbereich, das heißt sowohl mit dem unteren ersten Teilbereich als auch mit dem oberen ersten Teilbereich, kraftschlüssig verbunden und geeignet, auf diesen eine von dem zweiten Abstützbereich weg gerichtete Schubkraft auszuüben. Durch einen zweiteiligen ersten Abstützbereich kann die von der Stellvorrichtung ausgeübte Schubkraft effektiver auf das Körperskelett übertragen werden. Dadurch wird außerdem der Tragekomfort erhöht, da sich der durch die Abstützung auf Oberschenkel und gegebenenfalls Sitzbein ausgeübte Druck auf eine größere Körperfläche verteilt. Durch zweiteilige erste und zweite Abstützbereiche kann generell eine größere Schubkraft auf das erste und das zweite Körperteil übertragen werden, wodurch eine stärkere Entlastung bzw. Distraktion des Gelenks erreichbar ist.

Wie das zweite Halteteil kann das erste Halteteil der Orthese auch mehrteilig ausgebildet sein. Vorzugsweise weist das erste Halteteil dann eine Oberschenkelhülse und einen Sitzring auf, die zueinander beweglich oder starr miteinander verbunden sind. In diesem Fall kann sich ein unterer erster Teilbereich des ersten Abstützbereiches an der Oberschenkelhülse und oberer erster Teilbereich des ersten Abstützbereichs am Sitzring befinden. Oberschenkelhülse und Sitzring können vorteilhafterweise über ein Orthesengelenk zueinander um eine Achse des Orthesengelenks drehbar verbunden sein. Dies verbessert die Beweglichkeit der Orthese und damit die Bewegungsfreiheit des Patienten bei gleichzeitig effektiver Kraftübertragung durch zwei Abstütz-Teilbereiche.

Der Grad der Gelenksentlastung bei der Distraktion des Knie- und/oder Sprunggelenks kann vorzugsweise derart gewählt sein, dass sich das Gelenk bei einer Belastung des entsprechenden Beins mit dem Körpergewicht, beispielsweise in der Belastungsphase beim Gehen, in einer natürlichen und/oder entlasteten Stellung befindet und bei einer Nichtbelastung des Beins, beispielsweise in der Schwungphase beim Gehen, in einer entlasteten und/oder auseinandergezogenen Stellung befindet.

Die erfindungsgemäße Orthese kann ferner dahingehend ausgestaltet sein, dass sie für eine Distraktion eines Hüftgelenks verwendbar ist. In diesem Fall ist das erste Halteteil zur Aufnahme eines Rumpfes und das zweite Halteteil zur Aufnahme eines Oberschenkels geeignet, so dass mittels der Orthese eine zumindest zeitweise entlastete und/oder auseinandergezogene Stellung eines Hüftgelenks herbeiführbar ist. Das erste Halteteil ist dann bevorzugt als Korsett ausgebildet.

In einer besonders bevorzugten Ausgestaltung der Orthese sind das erste und das zweite Halteteil über mindestens ein Orthesengelenk um eine Drehachse des Orthesengelenks zueinander drehbar verbunden. Durch das Orthesengelenk bleibt das Körpergelenk in der durch die Orthese vorgegebenen Stellung beweglich. Die Beweglichkeit des Körpergelenks während des Tragens der Orthese kann die Heilung des Körpergelenks beschleunigen. Außerdem wird dem Patienten somit während der Behandlung des Gelenks mit der Orthese ein zumindest weitgehend normaler Gang ermöglicht.
Das Orthesengelenk weist vorzugsweise eine obere und eine untere Gelenkschiene auf, wobei die obere Gelenkschiene mit dem ersten Halteteil verbunden ist und die untere Gelenkschiene mit der Stellvorrichtung verbunden ist.

Das Orthesengelenk kann auch mittels einer Sperreinrichtung feststellbar sein, so dass die Halteteile in einem festen Winkel bezüglich der Drehachse des Orthesengelenks zueinander fixierbar sind. Eine Feststellung des Orthesengelenks ist besonders vorteilhaft, wenn das Körpergelenk ruhig gestellt werden soll, das heißt, wenn keine Bewegung des Körpergelenks erwünscht ist, wie z.B. bei entzündlichen Prozessen oder postoperativen Zuständen.

Anstelle eines Orthesengelenks kann das erste Halteteil auch über eine Verbindungsschiene mit dem zweiten Halteteil verbunden sein.

In einer bevorzugten Ausführungsform weist die Stellvorrichtung mindestens eine Linearführung und/oder eine Gasdruckfeder auf, die gegebenenfalls miteinander gekoppelt sind. Die Linearführung weist vorzugsweise eine Führungsschiene und eine in der Führungsschiene parallel zur Führungsschiene verschiebbar gelagerte Gleitschiene auf. Die Führungsschiene kann mit dem ersten oder dem zweiten Halteteil fest verbunden sein, während die Gleitschiene mit Ihrem dem Orthesengelenk oder der Verbindungsschiene zugewandten Ende mit der oberen oder unteren Gelenkschiene oder mit der Verbindungsschiene verbunden ist. Mit ihrem dem Orthesengelenk oder der Verbindungsschiene abgewandten Ende ist die Gleitschiene gegebenenfalls mit der Gasdruckfeder gekoppelt. Die Gasdruckfeder kann mit dem ersten oder dem zweiten Abstützbereich des ersten oder zweiten Halteteils, je nachdem an welchem Halteteil auch die Linearführung angebracht wäre, verbunden sein. Die Gasdruckfeder kann derart eingerichtet sein, dass diese einen Druck auf den ersten oder zweiten Abstützbereich sowie auf die Gleitschiene ausüben kann. Durch den Druck kann sich die Gleitschiene relativ zur Führungsschiene verschieben und dadurch wiederum eine Schubkraft über das Orthesengelenk oder die Verbindungsschiene auf den zweiten bzw. ersten Abstützbereich ausüben.

Die Stellvorrichtung kann alternativ auch eine Spiralfeder, eine Zahnstange, eine Lochschiene, eine Gewindestange, eine Klemmvorrichtung, eine Rastervorrichtung und/oder elektrische oder mechanische Umlaufzüge zur Einstellung der Schubkraft auf den ersten und den zweiten Abstützbereich aufweisen.

Alternativ kann die Stellvorrichtung auch einen elektrisch gesteuerten Antrieb umfassen, der eine elektrisch gesteuerte und/oder automatische Ausübung der Schubkraft auf das erste und das zweite Halteteil ermöglicht. Der elektrisch gesteuerte Antrieb kann beispielsweise ein Schrittmotor, ein Spindelantrieb oder ein Linearmotor sein.

Die Stellvorrichtung kann ferner so ausgebildet sein, dass frei wählbare Kraftbeträge zwischen dem ersten und dem zweiten Halteteil innerhalb eines Bereichs zwischen einer minimalen und einer maximalen Schubkraft kontinuierlich oder in Stufen einstellbar sind.

Die Halteteile der Orthese weisen vorzugsweise Kunststoff oder Kohlefasermaterial auf oder bestehen daraus. Die Stellvorrichtung weist bevorzugt Metall oder Glas auf oder besteht daraus.

Auch ein Verfahren zum Herbeiführen einer zumindest zeitweise entlasteten und/oder auseinandergezogenen Stellung eines Körpergelenks mittels einer vorstehend beschriebenen Orthese ist offenbart, wobei mittels der Stellvorrichtung eine vorgegebene Schubkraft auf den ersten und den zweiten Abstützbereich eingestellt und ausgeübt wird.

Eine erfindungsgemäße Orthese wird zur Behandlung von Arthrose, von Pseudoarthrose und/oder von Pseudoarthrose-ähnlichen Zuständen, zur postoperativen Nachbehandlung des Kniegelenks und/oder Sprunggelenks, zur Ausheilung, zur Behandlung von Entzündungen und/oder Schmerzen sowie zur Achsenkorrektur verwendet.

Im Folgenden werden Beispiele einer erfindungsgemäßen Orthese anhand von Figuren beschrieben. Dabei werden verschiedene erfindungswesentliche oder vorteilhafte Elemente im Rahmen dieser Beispiele genannt, wobei auch einzelne dieser Elemente als solche zur Weiterbildung der Erfindung - auch herausgelöst aus dem Kontext der Beispiele - verwendet werden können. Ferner entsprechen in den Figuren gezeigte gleiche oder ähnliche Elemente gleichen oder ähnlichen Bezugszeichen.

Es zeigen
- Figur 1: eine Seitenansicht einer erfindungsgemäßen Orthese,
- Figur 2: eine Vorderansicht einer erfindungsgemäßen Orthese,
- Figur 3: eine bevorzugte Verteilung der Durchgangslöcher (Ausnehmungen) an einem Oberschenkel anhand eines schematischen Oberschenkelquerschnittes und
- Figur 4: eine bevorzugte Verteilung der Durchgangslöcher (Ausnehmungen) an einem Oberschenkel wie in Figur 3 anhand verschiedener schematischer Ansichten eines Oberschenkels.

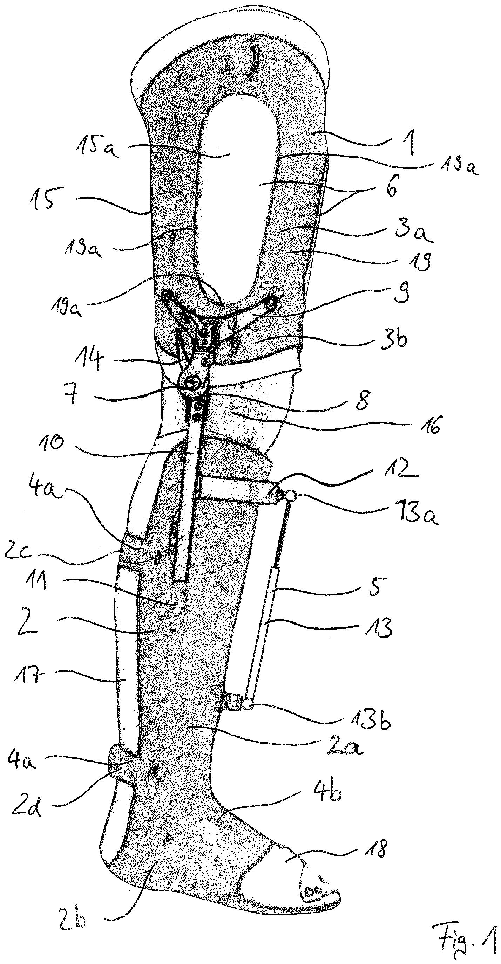

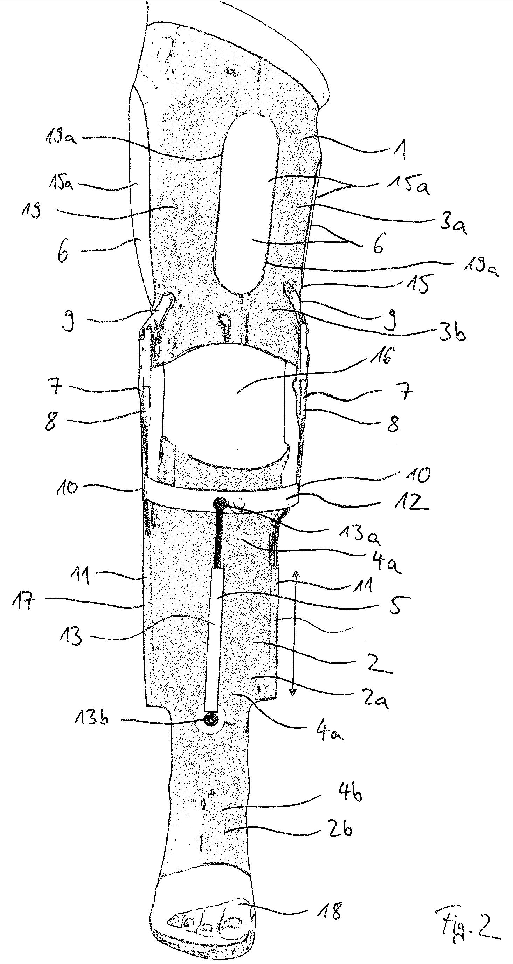

Figur 1 und Figur 2 zeigen eine Seiten- und eine Vorderansicht einer erfindungsgemäßen Orthese, die an ein Bein angelegt ist, zur Distraktion eines Kniegelenks 16. Die Orthese weist ein zylindermantelförmiges erstes Halteteil 1 auf, das an einen Oberschenkel 15 angelegt ist, sowie ein zweites Halteteil 2, das an einen Unterschenkel 17 angelegt ist. Das erste Halteteil 1 weist lediglich eine Oberschenkelhülse auf, während das zweite Halteteil 2 eine Unterschenkelhülse 2a zur Aufnahme des Unterschenkels 17 mit einer Ringfassung 2b zur Aufnahme eines Fußes 18 aufweist.

Im mittleren Bereich des ersten Halteteils 1 befindet sich ein oberer erster Abstützbereich 3a, in dem entlang des Umfangs längliche Durchgangslöcher 6 angeordnet sind. Die länglichen Durchgangslöcher 6 sind oval geformt und erstrecken sich in etwa über zwei Drittel der Länge des zylindermantelförmigen ersten Halteteils 1, wobei ihre Längsachsen in etwa parallel zur Längsachse des zylindermantelförmigen ersten Halteteils 1 ausgerichtet sind. Entlang des Umfangs des ersten Halteteils 1 sind die Durchgangslöcher 6 durch Stege 19 voneinander getrennt. Die Durchgangslöcher 6 weisen zu dem von den Durchgangslöchern 6 überdeckten Gewebe 15a zugewandte Innenkanten 19a auf, die in einem Querschnitt senkrecht zur Längsausdehnung des ersten Halteteils 1 konvex geformt sind. Mit dem dem zweiten Halteteil 2 zugewandten unteren Bereich der Innenkanten 19a der Durchgangslöcher 6 stützt sich das erste Halteteil 1 an dem von den Durchgangslöchern 6 überdeckten Gewebe 15a des Oberschenkels 15 ab. In seinem unteren, also dem zweiten Halteteil 2 zugewandten, Bereich weist das erste Halteteil 1 zusätzlich einen entlang des Umfangs des ersten Halteteils 1 umlaufenden unteren ersten Abstützbereich 3b auf, in dem sich das erste Halteteil 1 an einer Verbreiterung des Oberschenkelknochens im Bereich des Kniegelenks 16 abstützt.

Die Unterschenkelhülse 2a des zweiten Halteteils 2 ist entlang ihrer gesamten Länge im vorderen Bereich, also im Bereich des Schienbeins, bis zu den Seiten hin geschlossen ausgebildet. Im oberen hinteren Bereich, also im Bereich der Wade, ist die Unterschenkelhülse 2a lediglich durch einen oberen Steg 2c auf Höhe des Wadenmuskels und durch einen unteren Steg 2d auf Höhe des Sprunggelenks geschlossen. Zum Fuß 18 hin schließt sich an die Unterschenkelhülse 2a die Ringfassung 2b an, die den Fuß 18 am Fußrücken, an den Seiten sowie an der Sohle überdeckt. Das zweite Halteteil 2 weist einen oberen zweiten Abstützbereich 4a am oberen Steg 2c, mit dem sich das zweite Halteteil 2 am Wadenmuskel des Unterschenkels 17 abstützt, sowie am unteren Steg 2d, mit dem sich das zweite Halteteil 2 am Sprunggelenk abstützt, auf. Ein unterer zweiter Abstützbereich 4b befindet sich an der Ringfassung 2b im Bereich des Fußrückens, in dem sich das zweite Halteteil 2 am Fußrücken des Fußes 18 abstützt.

Das erste 1 und das zweite Halteteil 2 sind über seitlich an der Orthese angeordnete Orthesengelenke 7 und eine Stellvorrichtung 5 miteinander beweglich verbunden. Die Orthesengelenke 7 weisen jeweils obere Gelenkschienen 9 auf, deren den Orthesengelenken 7 abgewandte Enden gabelförmig ausgebildet sind und beidseits von den Orthesengelenken 7 zugewandten unteren Enden von seitlich angeordneten Durchgangslöchern 6 am ersten Halteteil 1 befestigt sind. Die Orthesengelenke 7 weisen ferner untere Gelenkschienen 8 auf, die sich von den Orthesengelenken 7 kommend bis in etwa zur dem ersten Halteteil 1 zugewandten Oberkante des zweiten Halteteils 2 erstrecken. An diesen unteren Enden sind die unteren Gelenkschienen 8 mit Gleitschienen 10 starr verbunden. Diese Gleitschienen 10 sind Teil der Stellvorrichtung 5. Die Stellvorrichtung 5 weist ferner beidseits der Unterschenkelhülse 2a Führungsschienen 11 auf, in denen die Gleitschienen 10 in Richtung der Längsausdehnung der Unterschenkelhülse 2a verschiebbar gelagert sind. Die Führungsschienen 11 sind fest mit der Unterschenkelhülse 2a verbunden. Die Gleitschienen 10 auf beiden Seiten der Unterschenkelhülse 2a sind durch einen querlaufenden Verbindungsbügel 12 starr miteinander verbunden. Die Stellvorrichtung 5 verfügt außerdem über eine Gasdruckfeder 13, die auf der Vorderseite der Unterschenkelhülse 2a in Längsrichtung der Unterschenkelhülse 2a angeordnet ist. Das dem ersten Halteteil 1 zugewandte obere Ende 13a der Gasdruckfeder 13 ist dabei mit dem Verbindungsbügel 12 verbunden. Das untere, der Ringfassung 2b zugewandte Ende der Gasdruckfeder 13 ist an der Unterschenkelhülse 2a befestigt.

Die Gasdruckfeder 13 übt eine konstante Schubkraft auf den zweiten Abstützbereich der 4a, 4b des zweiten Halteteils 2 einerseits und auf den Verbindungsbügel 12 andererseits auf. Über den Verbindungsbügel 12 wird die Schubkraft auf die Gleitschienen 10 beidseits der Unterschenkelhülse 2a übertragen. Je nach ausgeübter Schubkraft werden die Gleitschienen 10 in den Führungsschienen 11 in Richtung des Kniegelenks 16 aus den Führungsschienen 11 herausgeschoben. Diese Schubkraft wird wiederum über die Orthesengelenke 7 und die oberen Gelenkschienen 9 auf den ersten Abstützbereich 3a, 3b des ersten Halteteils 1 übertragen. Durch die Abstützung des zweiten Halteteils 2 am Fußrücken, an prominenten Stellen der Unterschenkelknochen im Bereich des Sprunggelenks sowie an der Wade im zweiten Abstützbereich 4a, 4b und des ersten Halteteils 1 an prominenten Stellen der Oberschenkelknochen im Bereich des Knies 16 sowie am Gewebe 15a des Oberschenkels 15 mithilfe der Durchgangslöcher 6 im ersten Abstützbereich 3a, 3b kommt es je nach von der Gasdruckfeder 13 ausgeübter Schubkraft zu einer Entlastung oder einer auseinandergezogenen Stellung (Distraktion) des Kniegelenks 16.

Durch die Orthesengelenke 7 wird selbst während der Distraktion eine Bewegung des Kniegelenks 16 ermöglicht. Falls eine Bewegung des Kniegelenks 16 nicht erwünscht ist, können die Orthesengelenke 7 über an den Orthesengelenken 7 angeordnete Sperrhebel 14 versteift werden.

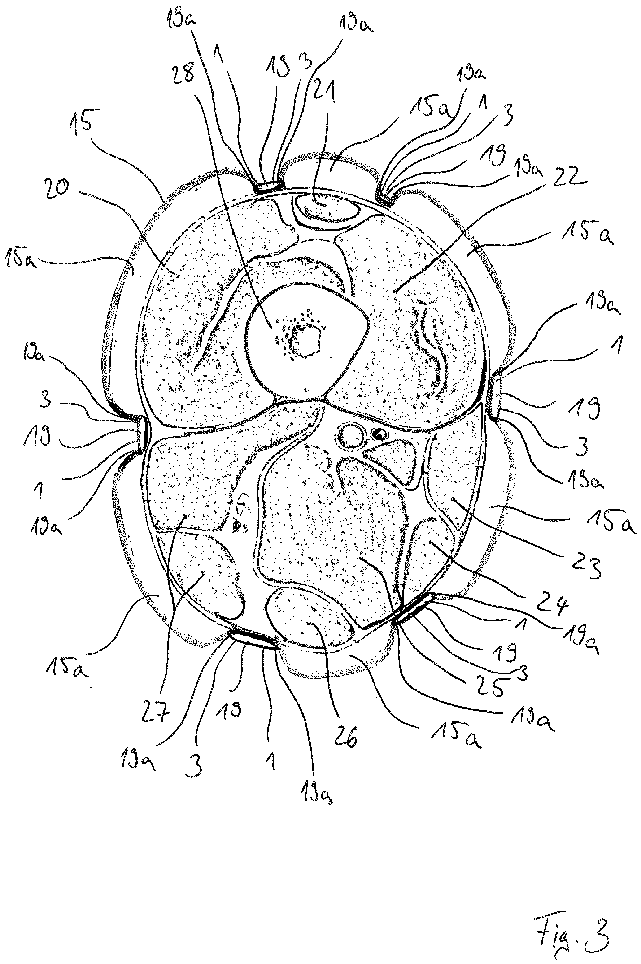

Figur 3 zeigt einen Querschnitt eines Oberschenkels 15 senkrecht zur Längsausdehnung des Oberschenkels 15 mit einem Oberschenkelknochen 28 und mit um den Oberschenkelknochen 28 angeordneten Oberschenkelmuskeln 20-27, an den das Halteteil 1 der Orthese, wie in den Figuren 1 und 2 dargestellt ist, angelegt ist. Der gezeigte Querschnitt befindet sich im Bereich des oberen ersten Abstützbereiches 3a des ersten Halteteils 1. Wie schon in den Figuren 1 und 2 gezeigt weist der obere erste Abstützbereich 3a entlang des Umfangs des ersten Halteteils 1 sechs Durchgangslöcher 6 auf, die in Richtung des Umfangs durch die Stege 19 voneinander getrennt sind. Durch die Durchgangslöcher 6 wölbt sich das Gewebe 15a des Oberschenkels 15, z.B. die Haut, nach außen. Die Stege 19 weisen an ihren dem sich nach außen wölbenden Gewebe 15a zugewandten Seiten Kanten auf, die den Innenkanten 19a der Durchgangslöcher 6 entsprechen. Im Querschnitt senkrecht zur Längsausdehnung des ersten Halteteils 1 sind die Innenkanten 19a konvex geformt. Die Durchgangslöcher 6 sind entlang der Oberschenkelmuskeln 20-27 angeordnet.

Dabei ist ein Durchgangsloch 6 entlang des Musculus vastus lateralis 20, ein Durchgangsloch 6 entlang des Musculus rectus femoris 21, ein Durchgangsloch 6 entlang des Musculus vastus medialis 22, ein Durchgangsloch 6 entlang des Musculus sartorius 23 und des Musculus gracilis 24, ein Durchgangsloch 6 entlang des Musculus adductor magnus 25 und des Musculus semitendinosus 26 und ein Durchgangsloch 6 entlang des Musculus biceps femoris 27 angeordnet.

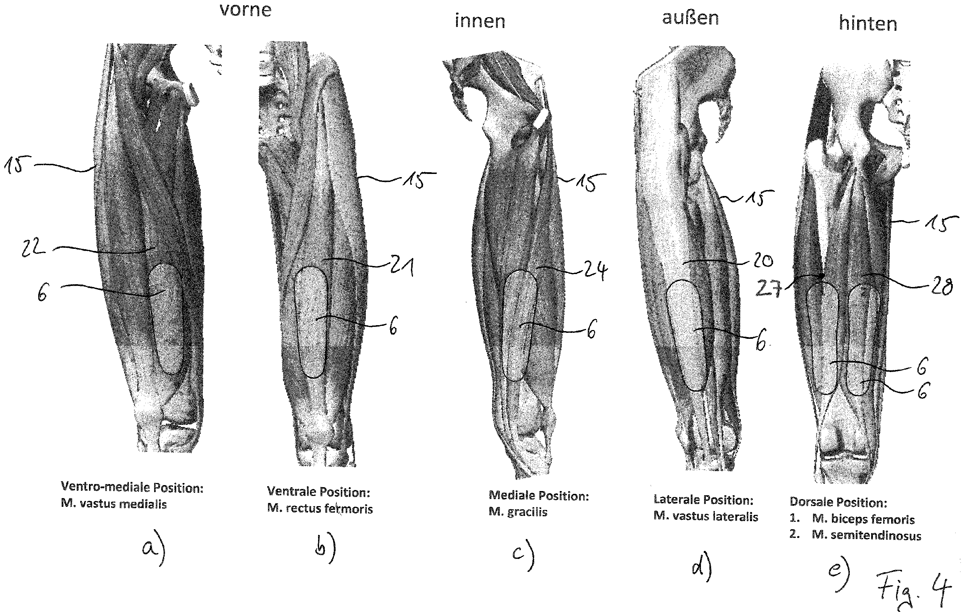

Figur 4 zeigt die Verteilung der Durchgangslöcher 6 wie in Figur 3 entlang von Oberschenkelmuskeln anhand von Vorder-, Seiten- und Rückansichten der Muskelstruktur eines Oberschenkels 15. Figur 4 a) zeigt eine Vorderansicht der Muskelstruktur an einem rechten Oberschenkel 15. Ein Durchgangsloch 6 ist entlang des Musculus vastus medialis 22 angeordnet. Figur 4 b) zeigt eine Vorderansicht der Muskelstruktur an einem linken Oberschenkel 15. Ein Durchgangsloch 6 ist entlang des Musculus rectus femoris 21 angeordnet. Figur 4 c) zeigt die Muskelstruktur an einer Innenseite eines linken Oberschenkels 15. Ein Durchgangsloch 6 ist entlang des Musculus gracilis 24 angeordnet. Figur 4 d) zeigt die Muskelstruktur an einer Außenseite eines linken Oberschenkels 15. Ein Durchgangsloch 6 ist entlang des Musculus vastus lateralis 20 angeordnet. Figur 4 e) zeigt eine Rückansicht der Muskelstruktur eines linken Oberschenkels 15. Ein Durchgangsloch 6 ist jeweils entlang des Musculus biceps femoris 27 und des Musculus semitendinosus 28 angeordnet.

In einer weiteren vorteilhaften Ausgestaltung der Erfindung weist der zweite Abstützbereich einen unteren zweiten und einen oberen zweiten Teilbereich zur Abstützung in zwei verschiedenen Bereichen längs des Unterschenkels und/oder am Fuß auf.

In einer weiteren vorteilhaften Ausgestaltung der Erfindung weist das zweite Halteteil eine Unterschenkelhülse und eine Fußfassung auf, welche zueinander beweglich oder starr miteinander verbunden sind.

In einer weiteren vorteilhaften Ausgestaltung der Erfindung ist die Fußfassung eine Ringfassung.

In einer weiteren vorteilhaften Ausgestaltung der Erfindung weist das zweite Halteteil eine Unterschenkelhülse auf, die in ihrem dem ersten Halteteil abgewandten Bereich eine Abstützplatte zur Abstützung des zweiten Halteteils am Fußrücken aufweist.

In einer weiteren vorteilhaften Ausgestaltung der Erfindung weist der erste Abstützbereich einen oberen ersten und einen unteren ersten Teilbereich zur Abstützung in zwei verschiedenen Bereichen längs des Oberschenkels und/oder am Sitzbein auf.

In einer weiteren vorteilhaften Ausgestaltung der Erfindung weist das erste Halteteil eine Oberschenkelhülse und einen Sitzring auf, die zueinander beweglich oder starr miteinander verbunden sind.

In einer weiteren vorteilhaften Ausgestaltung der Erfindung ist das erste Halteteil zur Aufnahme eines Rumpfes und das zweite Halteteil zur Aufnahme eines Oberschenkels geeignet und mittels der Orthese eine zumindest zeitweise entlastete und/oder auseinandergezogene Stellung eines Hüftgelenks herbeiführbar.

In einer weiteren vorteilhaften Ausgestaltung der Erfindung ist das erste Halteteil als Korsett ausgebildet.

In einer weiteren vorteilhaften Ausgestaltung der Erfindung weist die Stellvorrichtung eine Spiralfeder, eine Zahnstange, eine Lochschiene, eine Gewindestange, eine Klemmvorrichtung, eine Rastervorrichtung und/oder elektrische oder mechanische Umlaufzüge zur Einstellung der Schubkraft auf den ersten und den zweiten Abstützbereich auf.

In einer weiteren vorteilhaften Ausgestaltung der Erfindung weist die Stellvorrichtung einen elektrisch gesteuerten Antrieb, beispielsweise einen Schrittmotor, einen Spindelantrieb oder einen Linearmotor, auf, mittels dem die von der Stellvorrichtung auf den ersten und zweiten Abstützbereich ausgeübte Schubkraft ausübbar ist.

In einer weiteren vorteilhaften Ausgestaltung der Erfindung ist die Stellvorrichtung geeignet, frei wählbare Kraftbeträge innerhalb eines Bereichs zwischen einer minimalen und einer maximalen Schubkraft kontinuierlich oder in Stufen einzustellen.

In einer weiteren vorteilhaften Ausgestaltung der Erfindung ist das Orthesengelenk mittels einer Sperreinrichtung feststellbar, so dass die Halteteile in einem festen Winkel bezüglich der Drehachse des Orthesengelenks zueinander fixierbar sind.

In einer weiteren vorteilhaften Ausgestaltung der Erfindung weisen die Halteteile Kunststoff oder Kohlefasermaterial auf oder bestehen daraus.

In einer weiteren vorteilhaften Ausgestaltung der Erfindung weist die Stellvorrichtung Metall oder Glas auf oder besteht daraus.

Eine Verwendung einer erfindungsgemäßen Orthese zur Behandlung von Arthrose, Pseudoarthrose und/oder Pseudoarthrose-ähnlichen Zuständen, zur postoperativen Nachbehandlung des Kniegelenks und/oder Sprunggelenks, zur Ausheilung, zur Behandlung von Entzündungen und/oder Schmerzen oder zur Achsenkorrektur ist vorgesehen.

## Patentansprüche

1. Orthese zum Herbeiführen einer zumindest zeitweise entlasteten und/oder auseinandergezogenen Stellung eines Körpergelenks (16), wobei die Orthese mindestens ein erstes Halteteil (1) zur Aufnahme eines ersten Körperteils (15) und ein zweites Halteteil (2) zur Aufnahme eines mit dem ersten Körperteil (15) über das Körpergelenk (16) verbundenen zweiten Körperteils (17) aufweist, wobei
das erste Halteteil (1) mindestens einen ersten Abstützbereich (3a, 3b) aufweist, in dem das erste Halteteil (1) geeignet ist, sich in einer Längsrichtung des ersten Halteteils (1) und vom zweiten Halteteil (2) weg gerichtet am ersten Körperteil (15) abzustützen, und
das zweite Halteteil (2) mindestens einen zweiten Abstützbereich (4a, 4b) aufweist, in dem das zweite Halteteil (2) geeignet ist, sich in einer Längsrichtung des zweiten Halteteils (2) und vom ersten Halteteil (1) weg gerichtet am zweiten Körperteil (17) abzustützen,
und der erste (3a, 3b) und der zweite Abstützbereich (4a, 4b) über eine Stellvorrichtung (5) kraftschlüssig miteinander verbunden sind, wobei die Stellvorrichtung (5) geeignet ist, auf den ersten (3a, 3b) und den zweiten Abstützbereich (4a, 4b) jeweils entgegengesetzte Schubkräfte auszuüben,
**dadurch gekennzeichnet, dass**
der erste (3a, 3b) und/oder der zweite Abstützbereich (4a, 4b) auf einer Körperkontaktseite des ersten (1) bzw. zweiten Halteteils (2) eine oder mehrere Ausnehmungen (6) aufweisen, so dass das erste (1) bzw. das zweite Halteteil (2) geeignet sind, sich in Längsrichtung an einem Gewebe (15a) des ersten (15) bzw. des zweiten Körperteils (17) abzustützen.

2. Orthese nach einem der vorhergehenden Ansprüche, **dadurch gekennzeichnet, dass** die Ausnehmungen (6) Löcher mit vorzugsweise konvex geformten Innenkanten (19a) oder Ausbuchtungen sind.

3. Orthese nach einem der vorhergehenden Ansprüche, **dadurch gekennzeichnet, dass** die Ausnehmungen (6) entlang eines Umfangs der Halteteile (1, 2) angeordnet sind.

4. Orthese nach einem der vorhergehenden Ansprüche, **dadurch gekennzeichnet, dass** die Ausnehmungen (6) in einem Schnitt parallel und/oder in einem anderen Winkel zu einer Oberfläche der Halteteile (1, 2) länglich, oval und/oder konisch geformt sind, wobei Längsachsen der Ausnehmungen (6) parallel und/oder in einem anderen Winkel zur Längsrichtung der Halteteile (1, 2) ausgerichtet sind.

5. Orthese nach dem vorhergehenden Anspruch, **dadurch gekennzeichnet, dass** die Ausnehmungen (6) entlang von Muskeln (20-27) oder Bereichen mit stützendem Gewebe des ersten (15) und/oder zweiten Körperteils (17) am ersten (1) und/oder zweiten Halteteil (2) angeordnet sind.

6. Orthese nach einem der vorhergehenden Ansprüche, **dadurch gekennzeichnet, dass** das erste Halteteil (1) zur Aufnahme eines Oberschenkels (15) und/oder eines Rumpfes und das zweite Halteteil (2) zur Aufnahme eines Unterschenkels (17) und/oder Fußes (18) geeignet ist und mittels der Orthese eine zumindest zeitweise entlastete und/oder auseinandergezogene Stellung eines Kniegelenks (16) herbeiführbar ist.

7. Orthese nach dem vorhergehenden Anspruch, **dadurch gekennzeichnet, dass** der zweite Abstützbereich (4a, 4b) derart ausgebildet ist, dass das zweite Halteteil (2) geeignet ist, sich an einer prominenten Stelle eines Unterschenkelknochens im Bereich eines Sprunggelenks und/oder an einem Fußrücken abzustützen.

8. Orthese nach einem der Ansprüche 6 oder 7, **dadurch gekennzeichnet, dass** der zweite Abstützbereich (4a, 4b) derart ausgebildet ist, dass das zweite Halteteil (2) geeignet ist, sich an einer Wade abzustützen.

9. Orthese nach einem der Ansprüche 6 bis 8, **dadurch gekennzeichnet, dass** der erste Abstützbereich (3a, 3b) derart ausgebildet ist, dass das erste Halteteil (1) geeignet ist, den Oberschenkel (15) in der Nähe des Kniegelenks (16) weitgehend zu umgreifen und sich dadurch an mindestens einer prominenten Stelle eines Oberschenkelknochens (28) abzustützen.

10. Orthese nach einem der Ansprüche 6 bis 9, **dadurch gekennzeichnet, dass** der erste Abstützbereich (3a, 3b) derart ausgebildet ist, dass das erste Halteteil (1) geeignet ist, sich an einem Sitzbein abzustützen.

11. Orthese nach einem der vorhergehenden Ansprüche, **dadurch gekennzeichnet, dass** das erste (1) und das zweite Halteteil (2) über mindestens ein Orthesengelenk (7) zueinander um eine Drehachse des Orthesengelenks (7) drehbar verbunden sind.

12. Orthese nach dem vorhergehenden Anspruch, **dadurch gekennzeichnet, dass** das Orthesengelenk (7) eine obere (9) und eine untere Gelenkschiene (8) aufweist, wobei die obere Gelenkschiene (9) mit dem ersten Halteteil (1) verbunden ist und die untere Gelenkschiene (8) mit der Stellvorrichtung (5) verbunden ist.

13. Orthese nach einem der vorhergehenden Ansprüche, **dadurch gekennzeichnet, dass** die Stellvorrichtung (5) eine Linearführung und/oder eine Gasdruckfeder (13) aufweist, die gegebenenfalls miteinander gekoppelt sind.

## Claims

1. An orthosis for bringing about an at least temporarily load-relieved and/or uncompressed position of a body joint (16), wherein the orthosis has at least a first holding part (1) for receiving a first body part (15) and a second holding part (2) for receiving a second body part (17) connected to the first body part (15) via the body joint (16), wherein
the first holding part (1) has at least a first supporting region (3a, 3b) in which the first holding part (1) is suitable to be supported on the first body part (15) in a longitudinal direction of the first holding part (1) and directed away from the second holding part (2), and
the second holding part (2) has at least a second supporting region (4a, 4b) in which the second holding part (2) is suitable to be supported on the second body part (17) in a longitudinal direction of the second holding part (2) and directed away from the first holding part (1),
and the first (3a, 3b) and the second (4a, 4b) supporting region are connected together in non-positive manner via an adjustment device (5), wherein the adjustment device (5) is suitable for exerting opposing by means of the orthosis shear forces in each case on the first (3a, 3b) and the second supporting region (4a, 4b),
**characterised in that**
the first (3a, 3b) and/or the second supporting region (4a, 4b) on a body contact side of the first (1) or second holding part (2) respectively have one or more cutouts (6), so that the first (1) or the second holding part (2) respectively is suitable to be supported in the longitudinal direction on a tissue (15a) of the first (15) or the second body part (17) respectively.

2. An orthosis according to one of the preceding claims, **characterised in that** the cutouts (6) are holes with preferably convexly shaped inner edges (19a) or protrusions.

3. An orthosis according to one of the preceding claims, **characterised in that** the cutouts (6) are arranged along a periphery of the holding parts (1, 2).

4. An orthosis according to one of the preceding claims, **characterised in that** the cutouts (6) in a section parallel and/or at a different angle to a surface of the holding parts (1, 2) are formed to be elongate, oval and/or conical, with longitudinal axes of the cutouts (6) being oriented parallel and/or at a different angle to the longitudinal direction of the holding parts (1, 2).

5. An orthosis according to the preceding claim, **characterised in that** the cutouts (6) are arranged along muscles (20-27) or regions with supporting tissue of the first (15) and/or second body part (17) on the first (1) and/or second holding part (2).

6. An orthosis according to one of the preceding claims, **characterised in that** the first holding part (1) is suitable for receiving an upper leg (15) and/or a trunk and the second holding part (2) is suitable for receiving a lower leg (17) and/or foot (18), and an at least temporarily load-relieved and/or uncompressed position of a knee joint (16) can be brought about by means of the orthosis.

7. An orthosis according to the preceding claim, **characterised in that** the second supporting region (4a, 4b) is formed such that the second holding part (2) is suitable for being supported on a prominent point of a lower-leg bone in the region of an ankle joint and/or on a back of a foot.

8. An orthosis according to one of Claims 6 or 7, **characterised in that** the second supporting region (4a, 4b) is formed such that the second holding part (2) is suitable to be supported on a calf.

9. An orthosis according to one of Claims 6 to 8, **characterised in that** the first supporting region (3a, 3b) is formed such that the first holding part (1) is suitable largely to encompass the upper leg (15) in the vicinity of the knee joint (16) and thereby to be supported on at least one prominent point of a thigh bone (28).

10. An orthosis according to one of Claims 6 to 9, **characterised in that** the first supporting region (3a, 3b) is formed such that the first holding part (1) is suitable for being supported on an ischial bone.

11. An orthosis according to one of the preceding claims, **characterised in that** the first (1) and the second holding part (2) are connected by means of at least one orthotic joint (7) so as to be rotatable relative to each other about an axis of rotation of the orthotic joint (7).

12. An orthosis according to the preceding claim, **characterised in that** the orthotic joint (7) has an upper (9) and a lower joint rail (8), the upper joint rail (9) being connected to the first holding part (1) and the lower joint rail (8) being connected to the adjustment device (5).

13. An orthosis according to one of the preceding claims, **characterised in that** the adjustment device (5) has a linear guide and/or a gas pressure spring (13), which are optionally coupled together.

## Revendications

1. Orthèse pour l'obtention d'une position au moins temporairement soulagée et/ou étendue d'une articulation du corps (16), l'orthèse comprenant au moins une première partie de maintien (1) pour le logement d'une première partie du corps (15) et une deuxième partie de maintien (2) pour le logement d'une deuxième partie du corps (17) relié à la première partie du corps (15) par l'intermédiaire de l'articulation (16),
la première partie de maintien (1) comprenant au moins une première zone d'appui (3a, 3b), dans laquelle la première partie de maintien (1) est conçue pour s'appuyer dans une direction longitudinale de la première partie de maintien (1) et de manière opposée à la deuxième partie de maintien (2) contre la première partie du corps (15) et
la deuxième partie de maintien (2) comprenant au moins une deuxième zone d'appui (4a, 4b), dans laquelle la deuxième partie de maintien (2) est conçue pour s'appuyer dans une direction longitudinale de la deuxième partie de maintien (2) et de manière opposée à la première partie de maintien (1) contre la deuxième partie du corps (17),
et la première (3a, 3b) et la deuxième zone d'appui (4a, 4b) étant reliées entre elles par force par l'intermédiaire d'un dispositif de réglage (5), le dispositif de réglage (5) étant conçu pour exercer sur la première (3a, 3b) et la deuxième zone d'appui (4a, 4b) des forces de poussée opposées,
**caractérisée en ce que**
la première (3a, 3b) et la deuxième zone d'appui (4a, 4b) comprennent, sur un premier côté de contact corporel de la première (1) ou de la deuxième partie de maintien (2), un ou plusieurs évidements (6), de façon à ce que la première (1) ou de la deuxième partie de maintien (2) soient conçues pour s'appuyer, dans la direction longitudinale, contre un tissu (15a) de la première (15) ou de la deuxième partie du corps (17).

2. Orthèse selon l'une des revendications précédentes, **caractérisée en ce que** les évidements (6) sont des trous avec des arêtes internes (19a) ou des saillies de préférence convexes.

3. Orthèse selon l'une des revendications précédentes, **caractérisée en ce que** les évidements (6) sont disposés le long d'une circonférence des parties de maintien (1, 2).

4. Orthèse selon l'une des revendications précédentes, **caractérisée en ce que** les évidements (6) présentent en coupe une forme parallèle et/ou à un autre angle par rapport à une surface des parties de maintien (1, 2), une forme allongée, ovale et/ou conique, les axes longitudinaux des évidements (6) étant orientées parallèlement et/ou à un autre angle par rapport à la direction longitudinale des parties de maintien (1, 2).

5. Orthèse selon la revendication précédente, **caractérisée en ce que** les évidements (6) sont disposés le long de muscles (20 à 27) ou de zones avec un tissu de soutien de la première (15) et/ou de la deuxième partie du corps (17) sur la première (1) et/ou la deuxième partie de maintien (2).

6. Orthèse selon l'une des revendications précédentes, **caractérisée en ce que** la première partie de maintien (1) est conçue pour le logement d'une cuisse (15) et/ou d'un tronc et la deuxième partie de maintien (2) est conçue pour le logement d'une jambe (17) et/ou d'un pied (18) et l'orthèse permet d'obtenir une position au moins temporairement soulagée et/ou détendue d'une articulation de genou (16).

7. Orthèse selon la revendication précédente, **caractérisée en ce que** la deuxième zone d'appui (4a, 4b) est conçue de façon à ce que la deuxième partie de maintien (2) est conçue pour s'appuyer contre un endroit proéminent d'un os de la jambe au niveau d'une articulation de cheville et/ou d'un dos de pied.

8. Orthèse selon l'une des revendications 6 ou 7, **caractérisée en ce que** la deuxième zone d'appui (4a, 4b) est conçue de façon à ce que la deuxième partie de maintien (2) est conçue pour s'appuyer contre un mollet.

9. Orthèse selon l'une des revendications 6 à 8, **caractérisée en ce que** la première zone d'appui (3a, 3b) est conçue de façon à ce que la première partie de maintien (1) est conçue pour entourer largement la cuisse (15) à proximité de l'articulation du genou (16) et pour s'appuyer ainsi contre au moins un endroit proéminent d'un os de la cuisse (28).

10. Orthèse selon l'une des revendications 6 à 9, **caractérisée en ce que** la première zone d'appui (3a, 3b) est conçue de façon à ce que la première partie de maintien (1) est conçue pour s'appuyer contre un ischion.

11. Orthèse selon l'une des revendications précédentes, **caractérisée en ce que** la première (1) et la deuxième partie de maintien (2) sont reliées entre elles par l'intermédiaire d'au moins une articulation d'orthèse (7) de manière rotative autour d'un axe de rotation de l'articulation d'orthèse (7).

12. Orthèse selon la revendication précédente, **caractérisée en ce que** l'articulation d'orthèse (7) comprend un rail d'articulation supérieur (9) et un rail d'articulation inférieur (8), le rail d'articulation supérieur (9) étant relié avec la première partie de maintien (1) et le rail d'articulation inférieur (8) étant relié avec le dispositif de réglage (5).

13. Orthèse selon l'une des revendications précédentes, **caractérisée en ce que** le dispositif de réglage (5) comprend un guidage linéaire et/ou un ressort de compression à gaz (13), qui sont, le cas échéant, couplés entre eux.
